(19) Europäisches Patentamt
European Patent Office
Office européen des brevets

(11) EP 2 907 509 A1

(12) EUROPEAN PATENT APPLICATION
published in accordance with Art. 153(4) EPC

(43) Date of publication:
**19.08.2015 Bulletin 2015/34**

(21) Application number: **13845592.8**

(22) Date of filing: **11.10.2013**

(51) Int Cl.:
*A61K 9/54* (2006.01) *A61K 33/24* (2006.01)
*A61K 47/02* (2006.01) *A61K 47/34* (2006.01)
*A61K 49/00* (2006.01) *A61P 35/00* (2006.01)
*A61P 43/00* (2006.01) *B01J 13/14* (2006.01)
*C08G 69/02* (2006.01)

(86) International application number:
**PCT/JP2013/078400**

(87) International publication number:
**WO 2014/058079 (17.04.2014 Gazette 2014/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.10.2012 JP 2012227113**

(71) Applicants:
- **Teijin Limited**
  **Osaka-shi, Osaka 541-0054 (JP)**
- **The University of Tokyo**
  **Tokyo 113-8654 (JP)**

(72) Inventors:
- **KATAOKA, Kazunori**
  **Tokyo 113-8654 (JP)**
- **KISHIMURA, Akihiro**
  **Tokyo 113-8654 (JP)**
- **ANRAKU, Yasutaka**
  **Tokyo 113-8654 (JP)**
- **SAKAI, Mitsuru**
  **Hino-shi**
  **Tokyo 191-0065 (JP)**
- **OTA, Hideo**
  **Hino-shi**
  **Tokyo 191-0065 (JP)**
- **KONDO, Shiro**
  **Hino-shi**
  **Tokyo 191-0065 (JP)**
- **MOMOSE, Miho**
  **Hino-shi**
  **Tokyo 191-0065 (JP)**

(74) Representative: **Raynor, Stuart Andrew**
**J A Kemp**
**14 South Square**
**Gray's Inn**
**London WC1R 5JJ (GB)**

# (54) ELECTROSTATIC-BONDING-TYPE VESICLE INCLUDING METAL MICROPARTICLES

(57) A vesicle comprising fine metal particles, which has a membrane formed from both a first polymer of (a) or (b) shown below and a second polymer of (c) or (d) shown below (with the proviso that a combination of (b) and (d) is excepted), wherein partial crosslinking occurs between a cationic segment and an anionic segment in the above polymers:
First polymer:
(a) a block copolymer (I) having both an electrically non-charged hydrophilic segment and a cationic segment
(b) an amino acid polymer (I) having a cationic segment
Second polymer:
(c) a block copolymer (II) having both an electrically non-charged hydrophilic segment and an anionic segment
(d) an amino acid polymer (II) having an anionic segment.

Fig.1

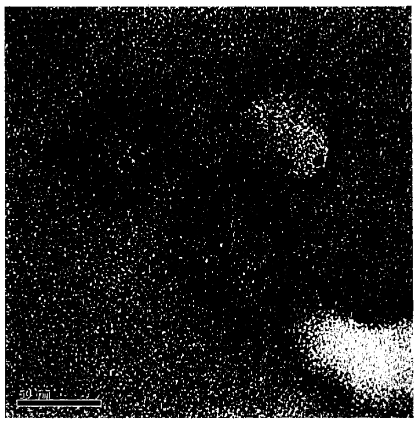

EP 2 907 509 A1

**Description**

TECHNICAL FIELD

The present invention relates to a vesicle comprising fine metal particles, which is formed from water-soluble and charged polymers.

BACKGROUND ART

It is known that polymers whose primary structure is precisely controlled may be spontaneously assembled to form a higher-order structure. Specific examples include structures such as micelles and vesicles. In the case of such structures formed by self-assembly of polymers, various types of molecules can be designed and may serve as structures having new functions in addition to characteristics inherent in the polymers. Utilization of such structures formed by self-assembly of polymers has been examined in various fields such as those of biomedicine and material science.

For example, Non-patent Document 1 discloses a vesicle made by self-assembly of a block copolymer having an electrically non-charged hydrophilic segment and a charged segment (e.g., polyethylene glycol (PEG)-polyanion) and a copolymer having an electric charge which is opposite to that of the charged segment (e.g., polycation). According to this technique, only by mixing two types of polymer aqueous solutions, a vesicle made of one electrostatically-bonded membrane with a uniform diameter of 100 to 400 nm can be prepared in a simple manner. Moreover, according to Patent Documents 1 and 2, only by mixing two types of polymer aqueous solutions, it is possible not only to prepare a vesicle in a simple manner, but also to obtain a vesicle stabilized through crosslinking.

On the other hand, there are some reports of fine metal particles being applied to the biomedical field and/or the optical field, etc. For example, in Patent Documents 3 and 4, gold nanorods are used as materials for novel spectroscopic analysis using near infrared light as a probe, are also used for imaging of tumor cells or the like through two-photon emission, and are further used for photothermal treatment based on photothermal conversion functions, which is designed to kill tumor cells or the like by the generated heat. As to self-assembling structures comprising fine metal particles, liposomes incorporating gold nanoparticles are used and evaluated for cellular uptake in Non-patent Document 2. Likewise, in Non-patent Document 3, colloidal gold-encapsulating liposomes are administered to mice, thereby confirming that colloidal gold is located around blood vessels in grafted tumor tissues, as observed by transmission electron microscopy (hereinafter expressed as "TEM").

However, Non-patent Document 1 makes no mention of an electrostatically bonded vesicle comprising fine metal particles, while Patent Document 1 presents water-dispersible metal nanoparticles as examples of a substance to be encapsulated within the hollow space of an empty vesicle, but there is no mention of a vesicle comprising metal nanoparticles inserted into its electrostatically bonded membrane (vesicle membrane). Patent Documents 3 and 4 report some cases where fine metal particles are used and applied to the biomedical field and/or the optical field, but they have no function of encapsulating a compound, unlike the vesicles appearing in Non-patent Document 1, Patent Document 1 and Patent Document 2. In Non-patent Documents 2 and 3, fine metal particles are encapsulated into liposomes to analyze cellular uptake and liposome behavior in mice at the particle level, but there is no description showing that the fine metal particles stably remain without being released from the liposomes.

CITATION LIST

PATENT DOCUMENTS

Patent Document 1: WO2011/145745
Patent Document 2: WO2012/014942
Patent Document 3: Japanese Laid-Open Patent Publication No. 2010-53111
Patent Document 4: Japanese Laid-Open Patent Publication No. 2011-63867


NON-PATENT DOCUMENTS

Non-patent Document 1: J. Am. Chem. Soc., 2010, 132(5), 1631-1636
Non-patent Document 2: nanomedicine, 2010, 6(1), 161-169
Non-patent Document 3: J. Electron Microsc. (Tokyo), 2011, 60(1), 95-99

SUMMARY OF INVENTION

TECHNICAL PROBLEM

The object of the present invention is to provide a vesicle comprising fine metal particles, which is formed from water-soluble and charged polymers, particularly a vesicle comprising fine metal particles in the vesicle membrane.

SOLUTION TO PROBLEM

As a result of extensive and intensive efforts made to solve the problem stated above, the inventors of the present invention have found not only that a vesicle comprising fine metal particles can be prepared in a simple manner, but also that these fine metal particles stably remain in the vesicle membrane without being released from the vesicle in an *in vivo* environment. These findings led to the completion of the present invention.

That is, the present invention relates to a vesicle comprising fine metal particles, which has a membrane formed from both a first polymer of (a) or (b) shown below and a second polymer of (c) or (d) shown below (with the proviso that a combination of (b) and (d) is excepted), wherein partial crosslinking occurs between cationic segment in the first polymer and anionic segment in the second polymer:

First polymer:

(a) a block copolymer (I) having both an electrically non-charged hydrophilic segment and a cationic segment
(b) an amino acid polymer (I) having a cationic segment

Second polymer:

(c) a block copolymer (II) having both an electrically non-charged hydrophilic segment and an anionic segment
(d) an amino acid polymer (II) having an anionic segment

ADVANTAGEOUS EFFECT OF INVENTION

The vesicle of the present invention can be present stably even in an *in vivo* environment and is capable of encapsulating a drug or the like within the vesicle membrane. In particular, a vesicle comprising fine metal particles in the vesicle membrane ensures stable holding of the fine metal particles.

BRIEF DESCRIPTION OF DRAWINGS

Figure 1 shows a TEM image of the vesicles prepared in Example 1 comprising colloidal gold intercalated therein.
Figure 2 shows a TEM image of the cell sections prepared in Example 2 after addition of the vesicles comprising colloidal gold intercalated therein.
Figure 3 shows a TEM image of the mouse liver sections prepared in Example 3 after intravenous administration of the vesicles comprising colloidal gold intercalated therein.
Figure 4 shows a TEM image of the vesicles prepared in Example 4 comprising colloidal palladium intercalated therein.
Figure 5 shows a TEM image of the vesicles prepared in Example 5 comprising colloidal platinum intercalated therein.
Figure 6 shows a TEM image of the vesicles prepared in Example 6 comprising gold nanorods intercalated therein.
Figure 7 shows a time-dependent plot of the blood level/dose ratio in mice obtained for vesicles comprising colloidal gold intercalated therein.
Figure 8 shows a time-dependent plot of the blood level/dose ratio in mice obtained for liposomes encapsulating colloidal gold therein.
Figure 9 shows a Raman spectrum for anti-Stokes and Stokes scattering in a sample which contains vesicles comprising colloidal gold intercalated therein and chloroform as a temperature marker. The solid line represents Au(+), and the dotted line represents Au(-).

DESCRIPTION OF EMBODIMENTS

Hereinafter, illustrative embodiments of the present invention will be described in detail.

1. Summary

The inventors of the present invention have found that a vesicle comprising fine metal particles can be prepared in a simple manner when two polymers including a positively-charged segment and a negatively-charged segment are used and mixed with fine metal particles. The inventors of the present invention have also found that the fine metal particles stably remain without being released from the vesicle in an *in vivo* environment. The present invention has been completed on the basis of these findings.

The vesicle of the present invention can be prepared without use of an organic solvent, and can be advantageously used in the biomaterial field and in drug delivery systems (DDS). Further, the vesicle of the present invention has a space (hollow) inside the vesicle membrane, and a large amount of a substance such as a compound can be encapsulated therein. Therefore, the vesicle of the present invention can be advantageously used, for example, as a delivery carrier for a substance in the body and a drug or as a fine reactor particle whose hollow serves as a reaction field of an enzyme. Moreover, the structure of the vesicle of the present invention can be stably maintained in the presence of saline or serum, and it is possible to impart various functions such as semi-permeability to the vesicle membrane. Therefore, the vesicle of the present invention can be advantageously used as a biomaterial or drug delivery system having excellent structure stability and environmental responsiveness.

The vesicle of the present invention not only has the above features as a vesicle, but also can be advantageously used for biomedical, material, optical and industrial purposes, e.g., as a material for novel spectroscopic analysis using near infrared light as a probe, for imaging of tumor cells or the like through two-photon emission or X-rays, etc., for photothermal treatment based on photothermal conversion functions, which is designed to kill tumor cells or the like by the generated heat, and further as a probe for TEM observation.

The term "vesicle" as used herein means a basic structure which has a hollow and is closed by a vesicle membrane.

Unless otherwise specified, the term "alkyl" or "alkoxy" as used herein as a group or a part of the group means that the group is a linear, branched or cyclic alkyl or alkoxy. Further, for example, "$C_{1-12}$" of "$C_{1-12}$ alkyl group" means that the carbon number of the alkyl group is 1 to 12.

Examples of the "$C_{1-12}$ alkyl group" in the present invention include methyl group, ethyl group, n-propyl group, isopropyl group, n-butyl group, sec-butyl group, tert-butyl group, n-pentyl group, n-hexyl group, decyl group and undecyl group. Examples of the "$C_{1-6}$ alkyl group" include methyl group, ethyl group, n-propyl group, isopropyl group, n-butyl group, sec-butyl group, tert-butyl group, n-pentyl group and n-hexyl group.

Unless otherwise specified, the term "aryl" as used herein means phenyl, naphthyl, anthnyl, pyrenyl or the like.

The term "halogen atom" as used herein means fluorine atom, chlorine atom, bromine atom or iodine atom.

The expression that the alkyl group is "optionally substituted" as used herein means that one or more hydrogen atoms on the alkyl group may be substituted with one or more substituents (which may be the same or different). It is apparent to those skilled in the art that the maximum number of substituents can be determined depending on the number of substitutable hydrogen atoms on the alkyl. Regarding groups other than the alkyl group, the expression "optionally substituted" is interpreted in the same way.

Substituents intended in the expression "optionally substituted" are selected from the group consisting of halogen atom, aryl group, hydroxyl group, amino group, carboxyl group, cyano group, formyl group, dimethylacetalized formyl group, diethylacetalized formyl group, $C_{1-6}$ alkoxycarbonyl group, $C_{2-7}$ acylamide group, tri-$C_{1-6}$ alkylsiloxy group (wherein $C_{1-6}$ alkyls may be the same or different), siloxy group and silylamino group.

2. Vesicle

One feature of the vesicle of the present invention lies in that it includes a vesicle membrane formed by the interaction of water-soluble and charged polymers.

The vesicle of the present invention has a vesicle membrane which is formed from a first polymer and a second polymer shown below (with the proviso that a combination of (b) and (d) is excepted). In this vesicle membrane, partial crosslinking occurs between cationic segment in the first polymer and anionic segment in the second polymer.

First polymer:

(a) a block copolymer (I) having both an electrically non-charged hydrophilic segment and a cationic segment
(b) an amino acid polymer (I) having a cationic segment

Second polymer:

(c) a block copolymer (II) having both an electrically non-charged hydrophilic segment and an anionic segment
(d) an amino acid polymer (II) having an anionic segment

However, such a vesicle membrane is preferably free from any sulfide structure such as a sulfide group.

Moreover, the outer and inner surfaces of the vesicle membrane in the present invention are preferably hydrophilic. That is, the vesicle membrane in the vesicle of the present invention has a three-layer structure consisting of an outer layer, an intermediate layer and an inner layer, preferably wherein the outer layer and the inner layer are each composed of the electrically non-charged hydrophilic segment and the intermediate layer is composed of the cationic and anionic segments which are partially crosslinked with each other. In other words, in the vesicle membrane composed of the first polymer and the second polymer in the vesicle of the present invention, it is preferred that the electrically non-charged hydrophilic segments of the first and second polymers are located on the outside of the vesicle membrane (inner layer, outer layer), while the cationic and anionic segments which are partially crosslinked with each other are located in the interior of the vesicle membrane (intermediate layer).

The form of the vesicle of the present invention is usually a spherical shape. The particle diameter of the vesicle of the present invention is not particularly limited as long as the vesicle has a hollow structure, but preferably 10 μm or less, and more preferably 50 nm to 1 μm.

The vesicle of the present invention is a vesicle in which a polyion complex (PIC) is formed in the intermediate layer. Therefore, the vesicle of the present invention may be sometimes referred to as "PICsome."

3. Segment

Hereinafter, segments which constitute the vesicle of the present invention will be described.

(1) Charged segment

The charged segment included in the first polymer and the charged segment included in the second polymer can be charged with mutually opposite electric charges. In the present invention, the charged segment included in the first polymer is a cationic segment, and the charged segment included in the second polymer is an anionic segment.

Further, in the present invention, when polyamine is used as the cationic segment, the polyamine can be positively-charged by acid addition thereto. The type of acid to be added is determined as appropriate according to use of the vesicle, etc.

According to a preferred embodiment of the present invention, the cationic segment of the first polymer is represented by the following formula (1):

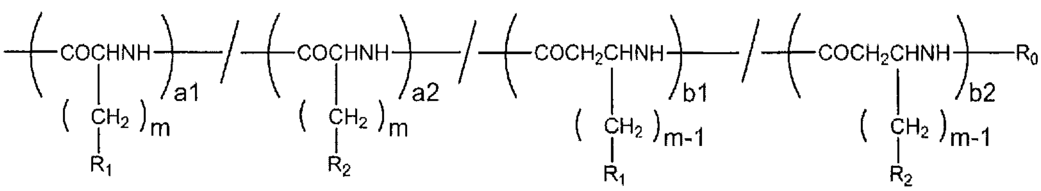

(1)

In the formula (1) above, $R_0$ represents a hydrogen atom, an acetyl group, a trifluoroacetyl group, an acryloyl group or a methacryloyl group, $R_1$ and $R_2$ each independently represent $-(CH_2)_3NH_2$ or $-CONH(CH_2)_S-X$, wherein s is an integer of 0 to 20 and X is $-NH_2$, a pyridyl group, a morpholyl group, a 1-imidazolyl group, a piperazinyl group, a 4-($C_{1-6}$ alkyl)-piperazinyl group, a 4-(amino $C_{1-6}$ alkyl)-piperazinyl group, a pyrrolidin-1-yl group, a N-methyl-N-phenylamino group, a piperidinyl group, a guanidino group, a diisopropylamino group, a dimethylamino group, a diethylamino group, $-(CH_2)_tNH_2$ or $-(NR_9(CH_2)_o)_pNHR_{10}$, wherein $R_9$ represents a hydrogen atom or a methyl group, $R_{10}$ represents a hydrogen atom, an acetyl group, a trifluoroacetyl group, a benzyloxycarbonyl group, -C(=NH)-$NH_2$ or a tert-butoxycarbonyl group, o is an integer of 1 to 15, p is an integer of 1 to 5, t is an integer of 0 to 15, m is 1 or 2, a1 and a2 are

each an integer of 0 to 5000, b1 and b2 are each an integer of 0 to 5000, and a1+a2+b1+b2 is 2 to 5000, and the symbol "/" means that the individual monomer units are sequenced in any order.

Further, according to a more preferred embodiment of the present invention, in the formula (1) above, X is $-NH_2$ or a guanidino group, s is an integer of 2 to 8, o is an integer of 1 to 10, $R_0$ is a hydrogen atom, a1 and a2 are each an integer of 0 to 200, b1 and b2 are each an integer of 0 to 200, and a1+a2+b1+b2 is 10 to 200.

In the present invention, when the first polymer forms an amino acid polymer (I) having a cationic segment, the cationic segment may be represented by the formula (1) above, and examples of the terminus opposite to Ro thereof include $-NH(CH_2)_{k-1}CH_3$ and $-NH-(CH_2)_{k-1}$-C(triple bond)CH (k is an integer of 1 or more), with $-NH(CH_2)_3CH_3$ being preferred.

In one embodiment of the present invention, the above-described amino acid polymer (I) is made of the cationic segment.

According to a preferred embodiment of the present invention, the anionic segment of the second polymer is represented by the following formula (2):

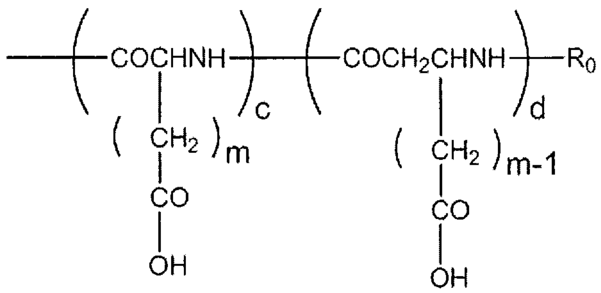

(2)

In the formula (2) above, $R_0$ represents a hydrogen atom, an acetyl group, a trifluoroacetyl group, an acryloyl group or a methacryloyl group, m is 1 or 2, c and d are each an integer of 0 to 5000, and c+d is 2 to 5000.

Further, according to a more preferred embodiment of the present invention, in the formula (2) above, $R_0$ is a hydrogen atom, c and d are each an integer of 0 to 200, and c+d is 10 to 200.

In the present invention, when the second polymer forms an amino acid polymer (II) having an anionic segment, the anionic segment may be represented by the formula (2) above, and examples of the terminus opposite to Ro thereof include - $NH(CH_2)_{w-1}CH_3$ and $-NH-(CH_2)_{w-1}$-C(triple bond)CH (w is an integer of 1 or more), with $-NH(CH_2)_3CH_3$ being preferred.

In one embodiment of the present invention, the above-described amino acid polymer (II) is made of the anionic segment.

(2) Electrically non-charged hydrophilic segment

The electrically non-charged hydrophilic segment is a polymer segment which is uncharged and hydrophilic. The term "electrically non-charged" as used herein means that the segment is neutral as a whole, as exemplified by a case where the segment does not have any positive or negative charge. Further, even if the segment has a positive/negative charge within its molecule, when a local effective charge density is not high and the charge of the segment is neutralized as a whole to the extent that it does not prevent the formation of the vesicle by self-assembly, it also corresponds to "electrically non-charged." The term "hydrophilic" means that the segment has solubility to an aqueous medium.

Examples of the electrically non-charged hydrophilic segment to be included in the block copolymer include polyalkylene glycol such as polyethylene glycol, and poly(2-oxazoline) such as poly(2-methyl-2-oxazoline), poly(2-ethyl-2-oxazoline), poly(2-n-propyl-2-oxazoline) and poly(2-isopropyl-2-oxazoline). Further examples include polysaccharide, polyvinyl alcohol, polyvinylpyrrolidone, polyacrylamide, polymethacrylamide, polyacrylic acid ester, polymethacrylic acid ester, poly(2-methacryloyloxyethylphosphorylcholine), a peptide, a protein and derivatives thereof, each having an isoelectric point of about 7. When the above-described electrically non-charged hydrophilic segment is included, the block copolymer can exist stably in an aqueous solution without assembly/precipitation, thereby efficiently constructing the vesicle. Moreover, when the vesicle is constructed with the block copolymers including the above-described electrically non-charged hydrophilic segment, the vesicle can maintain its structure stably in an aqueous solution.

According to a preferred embodiment of the present invention, the electrically non-charged hydrophilic segment of the first and second polymers is polyethylene glycol and/or poly(2-isopropyl-2-oxazoline), and preferably polyethylene glycol. Use of polyethylene glycol as the electrically non-charged hydrophilic segment is advantageous in imparting

biocompatibility to the vesicle.

When using polyethylene glycol as the electrically non-charged hydrophilic segment, the molecular weight of polyethylene glycol is preferably 500 to 15,000, and more preferably 1,000 to 5,000. Use of an electrically non-charged hydrophilic segment having the above-described molecular weight for the block copolymer is advantageous in forming the vesicle in preference to a micelle.

4. Block copolymer

(1) Block copolymer (I) having cationic segment

According to a preferred embodiment of the present invention, the block copolymer (I) having a cationic segment is represented by the following formula (3):

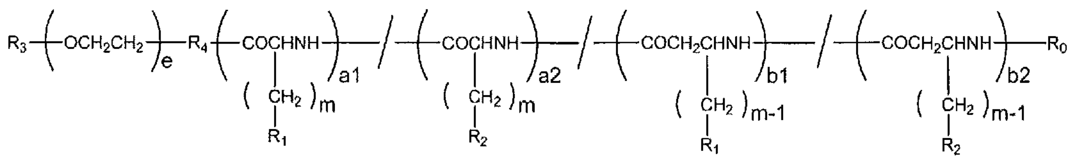

(3)

In the formula (3) above, $R_0$ represents a hydrogen atom, an acetyl group, a trifluoroacetyl group, an acryloyl group or a methacryloyl group, $R_1$ and $R_2$ each independently represent $-(CH_2)_3NH_2$ or $-CONH(CH_2)_S-X$, wherein s is an integer of 0 to 20 and X is $-NH_2$, a pyridyl group, a morpholyl group, a 1-imidazolyl group, a piperazinyl group, a 4-($C_{1-6}$ alkyl)-piperazinyl group, a 4-(amino $C_{1-6}$ alkyl)-piperazinyl group, a pyrrolidin-1-yl group, a N-methyl-N-phenylamino group, a piperidinyl group, a guanidino group, a diisopropylamino group, a dimethylamino group, a diethylamino group, $-(CH_2)_tNH_2$ or $-(NR_9(CH_2)_o)_pNHR_{10}$, wherein $R_9$ represents a hydrogen atom or a methyl group, $R_{10}$ represents a hydrogen atom, an acetyl group, a trifluoroacetyl group, a benzyloxycarbonyl group, $-C(=NH)-NH_2$ or a tert-butoxycarbonyl group, o is an integer of 1 to 15, p is an integer of 1 to 5, t is an integer of 0 to 15, $R_3$ represents a hydrogen atom or an optionally substituted linear or branched $C_{1-12}$ alkyl group, $R_4$ represents $-(CH_2)_gNH-$ and g is an integer of 0 to 5, e is an integer of 5 to 2500, m is 1 or 2, a1 and a2 are each an integer of 0 to 5000, b1 and b2 are each an integer of 0 to 5000, and a1+a2+b1+b2 is 2 to 5000, and the symbol "/" means that the individual monomer units are sequenced in any order.

Moreover, according to a more preferred embodiment of the present invention, in the formula (3) above, X is $-NH_2$ or a guanidino group, s is an integer of 2 to 8, o is an integer of 1 to 10, $R_0$ is a hydrogen atom, $R_3$ is a methyl group, a1 and a2 are each an integer of 0 to 200, b1 and b2 are each an integer of 0 to 200, and a1+a2+b1+b2 is 10 to 200, and e is an integer of 10 to 300.

(2) Block copolymer (II) having anionic segment

According to a preferred embodiment of the present invention, the block copolymer (II) having an anionic segment is represented by the following formula (4):

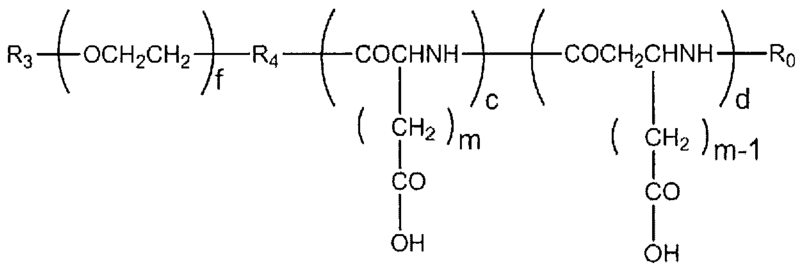

(4)

In the formula (4) above, $R_0$ represents a hydrogen atom, an acetyl group, a trifluoroacetyl group, an acryloyl group or a methacryloyl group, $R_3$ represents a hydrogen atom or an optionally substituted linear or branched $C_{1-12}$ alkyl group, $R_4$ represents $-(CH_2)_gNH-$ and g is an integer of 0 to 5, f is an integer of 5 to 2500, m is 1 or 2, c and d are each an integer of 0 to 5000, and c+d is 2 to 5000.

According to a more preferred embodiment of the present invention, in the formula (4) above, $R_0$ is a hydrogen atom, $R_3$ is a methyl group, c and d are each an integer of 0 to 200, and c+d is 10 to 200, and f is an integer of 10 to 300.

5. Crosslinking

In the vesicle membrane in the present invention, partial crosslinking occurs between cationic segment in the first polymer and anionic segment in the second polymer.

For crosslinking, in the presence of a suitable condensation agent, an amide bond may be formed, e.g., between the terminal amino group of the side chain of the cationic segment and the terminal carboxyl group of the side chain of the anionic segment, thereby crosslinking the segments. However, the positions for crosslinking in the cationic and anionic segments, the type of functional group used for crosslinking, and the mode of crosslinking are not limited to this embodiment. Moreover, crosslinking is not limited to between cationic segment and anionic segment, and the present invention also includes crosslinking between cationic segments or between anionic segments, and any combination thereof.

When using a crosslinking agent, the type of the crosslinking agent is not limited, and can be selected as appropriate depending on the intended use of the vesicle, the types of the first polymer and the second polymer, the types of other components of the membrane, etc. However, in terms of efficient crosslinking and enhancement of stability of a substance-encapsulating vesicle, it is preferred to use a crosslinking agent, which reacts with a charged group possessed by a charged segment of the first polymer or the second polymer (for example, a cationic group such as an amino group, or an anionic group such as a carboxyl group) and does not react with any encapsulated substance. Specific examples of the crosslinking agent include a crosslinking agent for crosslinking an amino group (e.g., glutaraldehyde, dimethyl suberimidate dihydrochloride (DMS), dimethyl 3,3'-dithiobispropionimidate (DTBP)) and a crosslinking agent for providing a crosslink by condensation of an amino group and a carboxyl group (e.g., 1-ethyl-3-(3-dimethylaminopropyl)carbodiimide (EDC)), with glutaraldehyde, EDC, etc. being preferred, and EDC being particularly preferred. One type of crosslinking agent may be used solely. Alternatively, 2 or more types of crosslinking agents may be used in any combination at any ratio.

When using the crosslinking agent, the amount thereof can be determined as appropriate by those skilled in the art depending on properties of the crosslinking agent, properties of groups to be crosslinked or the like. For example, in the case of a crosslinking agent which provides crosslinking by condensation of an amino group and a carboxyl group, the crosslinking agent can be used in an amount of 0.05 to 20 equivalents, preferably 0.1 to 20 equivalents, and for example, 0.1, 0.5, 1.0, 5.0 or 10 equivalents of the carboxyl group or amino group.

6. Preparation of a vesicle comprising fine metal particles

Although detailed information about, e.g., procedures for preparation of the vesicle of the present invention and optimal conditions for mixing between cationic segment and anionic segment is also disclosed in the patent documents mentioned above, a vesicle comprising fine metal particles as intended in the present invention may be prepared in a simple manner, for example, by adding fine metal particles to a mixed solution containing the first and second polymers, or alternatively, by adding fine metal particles to either or both of the first and second aqueous solutions, followed by preparing a mixed solution therefrom. In these cases, the inventors of the present invention have found that the fine metal particles selectively accumulate only in the vesicle membrane. However, even when the vesicle of the present invention comprises fine metal particles in the vesicle membrane, the fine metal particles cannot be prevented from being present at any site other than the vesicle membrane.

According to a preferred embodiment of the present invention, metals intended in the fine metal particles are not limited in any way as long as they may be present in the form of fine particles, and examples of such a metal include gold, silver, platinum, copper, nickel, palladium, iridium, rhodium and so on. The fine metal particles are required to have a size smaller than that of the vesicle, and if the fine metal particles have a spherical shape, their particle diameter is preferably 0.1 nm to 1000 nm, and more preferably 1 nm to 100 nm. If the fine metal particles have a rod shape, their longitudinal length is preferably 0.1 nm to 1000 nm, and more preferably 1 nm to 100 nm. The fine metal particles in the present invention may be of any shape, but their preferred shape is spherical or rod.

Moreover, the inventors of the present invention surprisingly have found that the thus prepared vesicles comprising fine metal particles are not decomposed *in vivo,* and the fine metal particles stably remain in a state being inserted into the vesicle membrane. In view of the foregoing, the vesicle of the present invention can be advantageously used for biomedical, material, optical and industrial purposes, e.g., as a material for novel spectroscopic analysis using near infrared light as a probe, for imaging of tumor cells or the like through two-photon emission or X-rays, etc., for photothermal treatment based on photothermal conversion functions, which is designed to kill tumor cells or the like by the generated heat, and further as a probe for TEM observation.

## EXAMPLES

### [Example 1]

<u>Preparation of vesicles comprising colloidal gold intercalated therein</u>

<Synthesis of anionic and cationic segments>

Anionic block copolymers PEG-poly(a,b-aspartic acid) (PEG-P(Asp); Mn of PEG = 2,000, DP (degree of polymerization) of P(Asp) = 75) and poly([5-aminopentyl]-a,b-aspartamide) (homo-P(Asp-AP); DP of P(Asp-AP) = 82) were synthesized as described in Anraku Y et al., J. Am. Chem. Soc., 2010,132(5), 1631-1636.

<Preparation of vesicles comprising colloidal gold intercalated therein>

Solutions of PEG-P(Asp) and homo-P(Asp-AP) synthesized as above in 10 mM phosphate buffer (0 mM NaCl, pH 7.4) were each prepared at a concentration of 1 mg/mL. The PEG-P(Asp) solution (3 mL) and the homo-P(Asp-AP) solution (4.2 mL) were mixed together and stirred for 2 minutes. PICsomes were identified by dynamic light scattering (DLS) analysis and were found to have an average particle diameter of 126.3 nm and a polydispersity index (PdI) of 0.066. To this prepared PICsome solution (3.5 mL), 0.4 mL of a colloidal gold solution (average particle diameter: 8 nm, Winered Chemical Co., Ltd., Japan) was added and stirred for 2 minutes. To crosslink the polyion complex (PIC) membrane of the resulting PICsomes, an EDC solution (10 mg/mL) was added in an amount of 0.3 equivalents relative to the -COO- side chain and reacted overnight at room temperature. The reaction solution was purified by ultrafiltration and then fluorescently labeled by addition of Cy3 Mono-reactive Dye Pack (Catalog No. PA23001, GE Healthcare) and purified by gel filtration with a PD-10 column (GE Healthcare) to prepare the desired vesicles comprising colloidal gold intercalated therein. According to DLS analysis, the vesicles were found to have an average particle diameter of 125.8 nm and a PdI of 0.02.

<Structural confirmation of vesicles comprising colloidal gold intercalated therein by TEM observation>

The thus prepared vesicles comprising colloidal gold intercalated therein were confirmed for their structure by TEM. Sections were prepared from the purified vesicles and provided for observation. Figure 1 shows their photograph observed by TEM. An image characteristic of the vesicular hollow structure was confirmed, and further colloidal gold was found to be accumulated only in the vesicle membrane and inserted thereinto. Moreover, the ultrafiltrate did not show a red color peculiar to colloidal gold, and also colloidal gold in a free state was not found in the TEM image, thus indicating that colloidal gold surprisingly has the property of concentrating in the vesicle membrane.

### [Example 2]

<u>Cellular uptake of vesicles comprising colloidal gold intercalated therein</u>

The human uterine cervical cancer-derived HeLa cell line was used as recipient cells. A PET film was located on the bottom of each well in a 24-well plate, and HeLa cells were seeded thereon and cultured in 10% FBS-containing DMEM medium at 37°C in the presence of 5% $CO_2$ to ensure adhesion of the cells onto each PET film. Then, the vesicles prepared in Example 1 comprising colloidal gold intercalated therein were added and further incubated for 24 hours. The HeLa cells/PET film were washed twice with phosphate buffered saline (PBS) buffer and then fixed with 2.5% glutaraldehyde/PBS, followed by TEM observation. Figure 2 shows the TEM image obtained. The vesicles comprising colloidal gold intercalated therein were observed to adhere onto the cell membrane surface and then to be taken up into the cells while being kept in this state. This indicates that the vesicles comprising colloidal gold intercalated therein can be used as an effective tool for analyzing the intracellular kinetics of the vesicles.

### [Example 3]

<u>Evaluation of *in vivo* stability of vesicles comprising colloidal gold intercalated therein</u>

<Evaluation of organ distribution in mice>

A solution of the vesicles purified in Example 1 comprising colloidal gold intercalated therein was administered to ICR mice via the tail vein and euthanized by excess anesthesia with ether after 1 hour of administration to collect their

livers, which were then fixed by being soaked in a 2.5% glutaraldehyde/PBS solution.

<Confirmation of the distribution state of vesicles by TEM observation>

Sections were prepared from the liver tissues fixed above and observed by TEM. Figure 3 shows the TEM image obtained. The vesicles comprising colloidal gold intercalated therein could be confirmed near the head of the arrow. The vesicles comprising colloidal gold intercalated therein were observed extensively in the liver in a state where their particles were not decomposed. This indicates that the vesicles comprising colloidal gold intercalated therein also stably remain under *in vivo* conditions and can be advantageously used as a probe for tracing the *in vivo* kinetics of the vesicles.

<Time course of blood levels in mice>

In the same manner as shown above, vesicles comprising colloidal gold intercalated therein were prepared from Cy5-labeled PEG-P(Asp), homo-P(Asp-AP), colloidal gold and EDC (10 equivalents), and ICR mice were administered with these vesicles via the tail vein and their blood was collected over time. The resulting plasma samples were measured for fluorescence to calculate the plasma concentration of the vesicles for each sample. Plasma samples obtained in the same manner were analyzed by ICP-AES to calculate the plasma gold concentration for each sample. Figure 7 shows a time-dependent plot of the blood level/dose ratio. The vesicles and colloidal gold were found to show similar time courses, thus indicating that the vesicles comprising colloidal gold intercalated therein allow the colloidal gold to stably remain without being released from the vesicles.

[Comparative Example 1]

Hydrogenated soy phosphatidylcholine, N-(carbonyl-methoxypolyethylene glycol 2000)-1,2-distearoyl-sn-glycero-3-phosphoethanolamine sodium salt and cholesterol were dissolved at a ratio (mol%) of 57:5:38 in a chloroform/methanol mixture, evaporated to remove the solvents and then dried under vacuum to obtain a lipid film, to which an aqueous colloidal gold solution (0.4 mg/mL) was then added for hydration. This mixture was treated in a bath-type ultrasonicator, and unencapsulated colloidal gold was removed by ultrafiltration. To the resulting liposome solution, 1,1'-dioctadecyltetramethyl indotricarbocyanine iodide was added at a final concentration of 50 μg/mL. This mixture was concentrated by ultrafiltration while removing the fluorescent substrate in a free state, and finally passed through a 0.45 μm filter to prepare fluorescently labeled liposomes encapsulating colloidal gold therein. The prepared liposomes were administered to ICR mice via the tail vein, and their plasma samples were measured for fluorescence and gold concentration. Figure 8 shows a time-dependent plot of the blood level/dose ratio. The time course of liposome concentration calculated from the results of fluorescence measurement was found to last for a long period of time, whereas the time course of colloidal gold concentration calculated from the results of ICP-AES analysis was found to disappear rapidly after administration. This indicates that colloidal gold is rapidly released from the liposomes in an *in vivo* environment.

[Example 4]

Preparation of vesicles comprising colloidal palladium intercalated therein

In place of the colloidal gold in Example 1, a colloidal palladium solution (average particle diameter: 43 nm, Winered Chemical Co., Ltd., Japan) was used to prepare vesicles comprising colloidal palladium intercalated therein. Upon DLS analysis, the vesicles were found to have an average particle diameter of 74.3 nm and a PdI of 0.19. Figure 4 shows a TEM (negative staining) image of the purified vesicles.

[Example 5]

Preparation of vesicles comprising colloidal platinum intercalated therein

In place of the colloidal gold in Example 1, a colloidal platinum solution (average particle diameter: 20 nm, Winered Chemical Co., Ltd., Japan) was used to prepare vesicles comprising colloidal platinum intercalated therein. Upon DLS analysis, the vesicles were found to have an average particle diameter of 143.3 nm and a PdI of 0.115. Figure 5 shows a TEM (negative staining) image of the purified vesicles.

[Example 6]

Preparation of vesicles comprising gold nanorods intercalated therein

In place of the colloidal gold in Example 1, an aqueous dispersion of gold nanorods (Dai Nippon Toryo, Co., Ltd., Japan) was used to prepare vesicles comprising gold nanorods intercalated therein. Upon DLS analysis, the vesicles were found to have an average particle diameter of 136.7 nm and a PdI of 0.046. Figure 6 shows a TEM (negative staining) image of the purified vesicles.

[Example 7]

Evaluation of CT contrast ability

Vesicles comprising colloidal gold intercalated therein were prepared from PEG-P(Asp), homo-P(Asp-AP), colloidal gold and EDC (10 equivalents) and evaluated for their CT contrast ability. The colloidal gold concentration in the prepared vesicles was 6 mg/mL. When measured by using 3D micro X-ray CT R_mCT (Rigaku Corporation, Japan), the prepared solution was found to have a CT value of 340 HT (assuming that water was set to 0 HT) and therefore confirmed to have the CT contrast ability. Because of being stable *in vivo,* the vesicles comprising colloidal gold intercalated therein can be expected for use as a CT contrast medium.

[Example 8]

Evaluation of photothermal effect

<Temperature measurement by Raman spectroscopy>

Laser-induced temperature rise in electrostatically bonded vesicles comprising fine metal particles was observed by Raman spectroscopy. A laser beam incident to a sample can be separated into Rayleigh scattering at the same frequency as the incident beam and Raman scattering at frequencies different from that of the incident beam. Further, Raman scattering is classified into Stokes scattering (a lower frequency component than the incident beam) and anti-Stokes scattering (a higher frequency component than the incident beam). Moreover, the temperature of the sample can be determined from the intensity ratio of anti-Stokes and Stokes scattered light according to the relational expression shown below.

$$\frac{I_s^{\,anti-Stokes}}{I_s^{\,Stokes}} = \left(\frac{\omega_0 + \omega_k}{\omega_0 - \omega_k}\right)^4 \exp\left(-\frac{h\omega_k}{2\pi kT}\right)$$

*h:* Planck constant, *k:* Boltzmann constant, *T:* absolute temperature,
$\omega_0+\omega_k$, $\omega_0-\omega_k$: angular frequencies of anti-Stokes and Stokes scattered light

As a Raman spectroscopic system, a JobinYvon T64000 system was used. The measurement system was set to the macro mode, and a spectrum was obtained for a sample in a test tube while stirring with a small magnetic stirrer. As an excitation laser, a semiconductor laser (wavelength: 785 nm, laser power: 170 mW) or an $Ar^+$ laser (wavelength: 514.5 nm, laser power: 1 W) was used. A single polychromator was used as a spectroscope, while a CCD multichannel detector was used as a detector. In addition, a notch filter for blocking the Rayleigh light was located upstream of the spectroscope.

Since the vesicles prepared in this example have no appropriate Raman scattering in the range to be measured, chloroform was used as a marker for temperature measurement. Chloroform had been confirmed to have no large absorption in the wavelength range (785, 514.5 nm) of the excitation laser in the Raman system.

Figure 9 shows a Raman spectrum for anti-Stokes and Stokes scattering in a sample which contains the vesicles comprising colloidal gold intercalated therein and chloroform as a temperature marker. The horizontal axis represents the Raman shift and the vertical axis represents the intensity of scattered light. The Raman shift refers to a difference in wave number between the excitation laser and the Raman scattered light, and has a value unique to each substance. The peaks at 260 $cm^{-1}$ and -260 $cm^{-1}$ shown in Figure 9 are the Stokes and anti-Stokes scattering peaks of chloroform.

<Vesicles comprising colloidal gold intercalated therein>

Using the 514.5 nm laser as an excitation source, the intensity ratio of anti-Stokes scattering and Stokes scattering was determined for vesicles comprising colloidal gold intercalated therein in the presence of chloroform. As a result, the intensity ratio was found to be 0.286. In the absence of the vesicles comprising colloidal gold intercalated therein, the intensity ratio was 0.235, thus indicating that addition of the vesicles induced a temperature rise.

<Vesicles comprising gold nanorods intercalated therein>

Using the 768 nm laser as an excitation source, the intensity ratio of anti-Stokes scattering and Stokes scattering was determined for vesicles comprising gold nanorods intercalated therein in the presence of chloroform. As a result, the intensity ratio was found to be 0.578. In the absence of the vesicles comprising gold nanorods intercalated therein, the intensity ratio was 0.543, thus indicating that addition of the vesicles induced a temperature rise.

INDUSTRIAL APPLICABILITY

The vesicle of the present invention comprising fine metal particles can stably maintain its structure *in vivo* and is useful in the biomedical, material, optical and other fields, e.g., as a biomaterial, as a delivery carrier for a substance in the body or a drug, as a fine reactor particle whose hollow serves as a reaction field of an enzyme, as a material for spectroscopic analysis, as for imaging of tumor cells or the like, as for photothermal therapy, and as a probe for TEM.

## Claims

**1.** A vesicle comprising fine metal particles, which has a membrane formed from both a first polymer of (a) or (b) shown below and a second polymer of (c) or (d) shown below (with the proviso that a combination of (b) and (d) is excepted), wherein partial crosslinking occurs between a cationic segment in the first polymer and an anionic segment in the second polymer:

First polymer:

(a) a block copolymer (I) having both an electrically non-charged hydrophilic segment and a cationic segment
(b) an amino acid polymer (I) having a cationic segment

Second polymer:

(c) a block copolymer (II) having both an electrically non-charged hydrophilic segment and an anionic segment
(d) an amino acid polymer (II) having an anionic segment.

**2.** The vesicle according to claim 1, wherein the fine metal particles are located in the membrane of the vesicle.

**3.** The vesicle according to claim 1 or 2, wherein the membrane has a three-layer structure consisting of an outer layer, an intermediate layer and an inner layer, wherein the outer layer and the inner layer are each composed of the electrically non-charged hydrophilic segment and the intermediate layer is composed of the cationic segment and the anionic segment.

**4.** The vesicle according to any one of claims 1 to 3, wherein the electrically non-charged hydrophilic segment is polyethylene glycol.

**5.** The vesicle according to any one of claims 1 to 4, wherein the metal in the fine metal particles is one or more selected from the group consisting of gold, silver, platinum, copper, nickel, palladium, iridium and rhodium.

**6.** The vesicle according to any one of claims 1 to 5, wherein the fine metal particles have a spherical or rod shape.

**7.** The vesicle according to any one of claims 1 to 6, wherein the cationic segment is represented by the following formula (1):

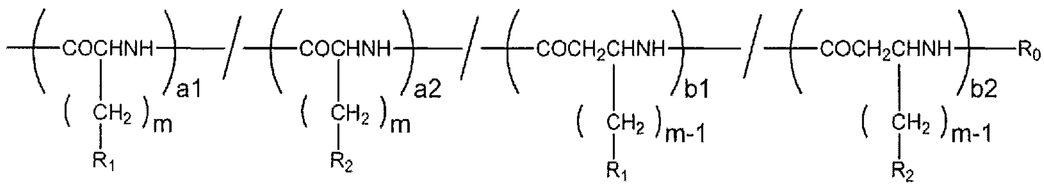

(1)

[wherein $R_0$ represents a hydrogen atom, an acetyl group, a trifluoroacetyl group, an acryloyl group or a methacryloyl group, $R_1$ and $R_2$ each independently represent-$(CH_2)_3NH_2$ or $-CONH(CH_2)_S$-X, wherein s is an integer of 0 to 20 and X is $-NH_2$, a pyridyl group, a morpholyl group, a 1-imidazolyl group, a piperazinyl group, a 4-($C_{1-6}$ alkyl)-piperazinyl group, a 4-(amino $C_{1-6}$ alkyl)-piperazinyl group, a pyrrolidin-1-yl group, a N-methyl-N-phenylamino group, a piperidinyl group, a guanidino group, a diisopropylamino group, a dimethylamino group, a diethylamino group, $-(CH_2)_tNH_2$ or - $(NR_9(CH_2)_o)_pNHR_{10}$, wherein $R_9$ represents a hydrogen atom or a methyl group, $R_{10}$ represents a hydrogen atom, an acetyl group, a trifluoroacetyl group, a benzyloxycarbonyl group, $-C(=NH)-NH_2$ or a tert-butoxycarbonyl group, o is an integer of 1 to 15, p is an integer of 1 to 5, t is an integer of 0 to 15, m is 1 or 2, a1 and a2 are each an integer of 0 to 5000, b1 and b2 are each an integer of 0 to 5000, and a1+a2+b1+b2 is 2 to 5000, and the symbol "/" means that the individual monomer units are sequenced in any order].

8. The vesicle according to claim 7, wherein X is $-NH_2$ or a guanidino group, s is an integer of 2 to 8, o is an integer of 1 to 10, $R_0$ is a hydrogen atom, a1 and a2 are each an integer of 0 to 200, b1 and b2 are each an integer of 0 to 200, and a1+a2+b1+b2 is 10 to 200.

9. The vesicle according to any one of claims 1 to 8, wherein the anionic segment is represented by the following formula (2):

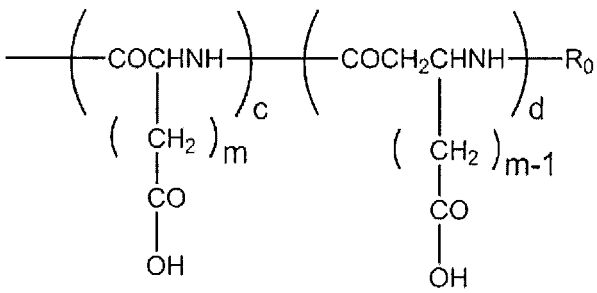

(2)

[wherein $R_0$ represents a hydrogen atom, an acetyl group, a trifluoroacetyl group, an acryloyl group or a methacryloyl group, m is 1 or 2, c and d are each an integer of 0 to 5000, and c+d is 2 to 5000].

10. The vesicle according to claim 9, wherein $R_0$ is a hydrogen atom, c and d are each an integer of 0 to 200, and c+d is 10 to 200.

11. The vesicle comprising fine metal particles according to any one of claims 1 to 6, wherein the block copolymer (I) is represented by the following formula (3):

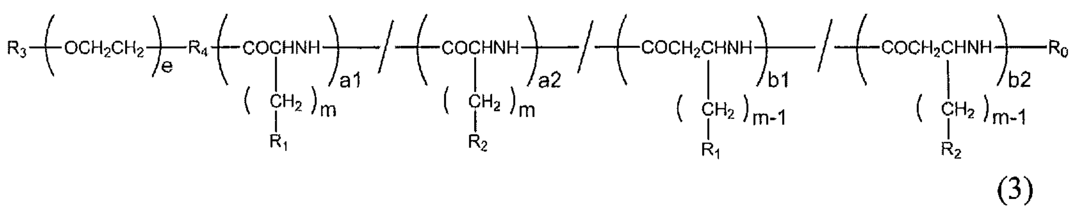

(3)

[wherein $R_0$ represents a hydrogen atom, an acetyl group, a trifluoroacetyl group, an acryloyl group or a methacryloyl group, $R_1$ and $R_2$ each independently represent-$(CH_2)_3NH_2$ or $-CONH(CH_2)_S-X$, wherein s is an integer of 0 to 20 and X is $-NH_2$, a pyridyl group, a morpholyl group, a 1-imidazolyl group, a piperazinyl group, a 4-($C_{1-6}$ alkyl)-piperazinyl group, a 4-(amino $C_{1-6}$ alkyl)-piperazinyl group, a pyrrolidin-1-yl group, a N-methyl-N-phenylamino group, a piperidinyl group, a guanidino group, a diisopropylamino group, a dimethylamino group, a diethylamino group, $-(CH_2)_tNH_2$ or-$(NR_9(CH_2)_o)_pNHR_{10}$, wherein $R_9$ represents a hydrogen atom or a methyl group, $R_{10}$ represents a hydrogen atom, an acetyl group, a trifluoroacetyl group, a benzyloxycarbonyl group, $-C(=NH)-NH_2$ or a tert-butoxycarbonyl group, o is an integer of 1 to 15, p is an integer of 1 to 5, t is an integer of 0 to 15, $R_3$ represents a hydrogen atom or an optionally substituted linear or branched $C_{1-12}$ alkyl group, $R_4$ represents - $(CH_2)_gNH-$ and g is an integer of 0 to 5, e is an integer of 5 to 2500, m is 1 or 2, a1 and a2 are each an integer of 0 to 5000, b1 and b2 are each an integer of 0 to 5000, and al+a2+bl+b2 is 2 to 5000, and the symbol "/" means that the individual monomer units are sequenced in any order].

**12.** The vesicle according to claim 11, wherein X is $-NH_2$ or a guanidino group, s is an integer of 2 to 8, o is an integer of 1 to 10, $R_0$ is a hydrogen atom, $R_3$ is a methyl group, a1 and a2 are each an integer of 0 to 200, b1 and b2 are each an integer of 0 to 200, and a1+a2+b1+b2 is 10 to 200, and e is an integer of 10 to 300.

**13.** The vesicle according to any one of claims 1 to 8, wherein the block copolymer (II) is represented by the following formula (4):

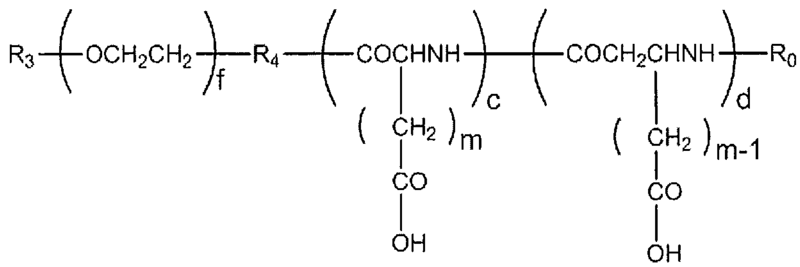

(4)

[wherein $R_0$ represents a hydrogen atom, an acetyl group, a trifluoroacetyl group, an acryloyl group or a methacryloyl group, $R_3$ represents a hydrogen atom or an optionally substituted linear or branched $C_{1-12}$ alkyl group, $R_4$ represents $-(CH_2)_gNH-$ and g is an integer of 0 to 5, f is an integer of 5 to 2500, m is 1 or 2, c and d are each an integer of 0 to 5000, and c+d is 2 to 5000].

**14.** The vesicle comprising fine metal particles according to claim 13, wherein Ro is a hydrogen atom, $R_3$ is a methyl group, c and d are each an integer of 0 to 200, and c+d is 10 to 200, and f is an integer of 10 to 300.

Fig.1

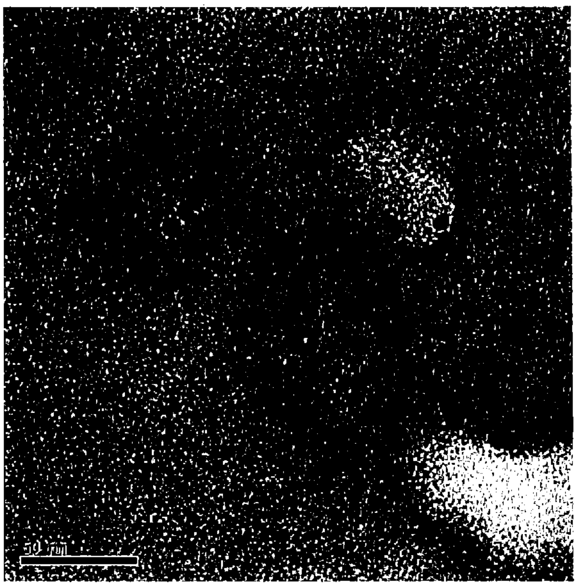

Fig.2

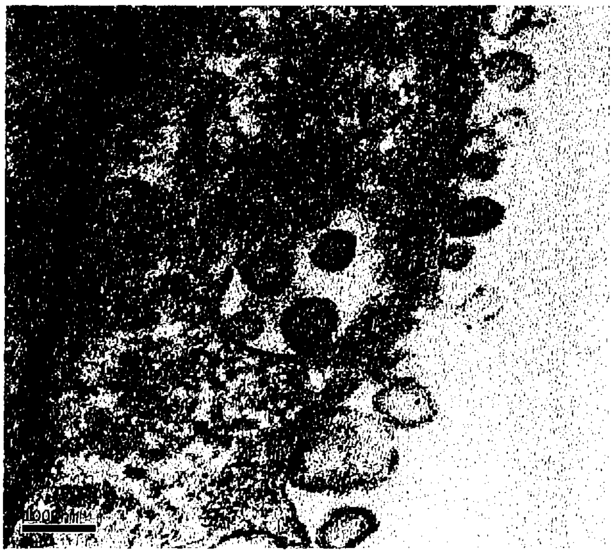

Fig.3

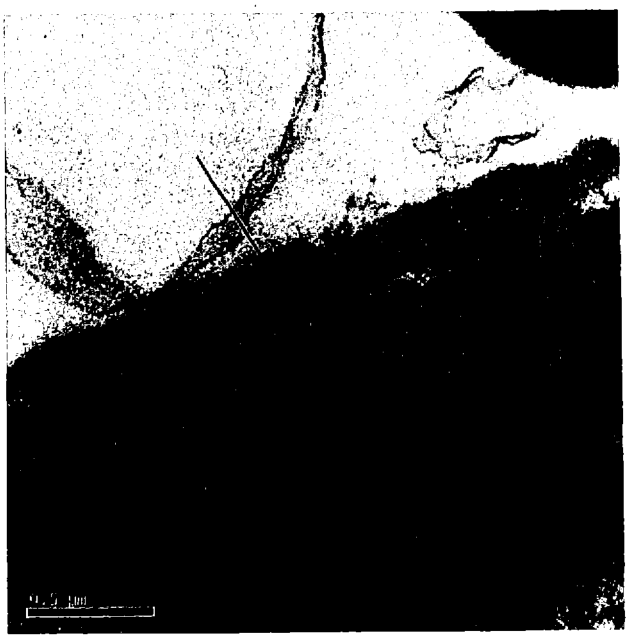


Fig.4

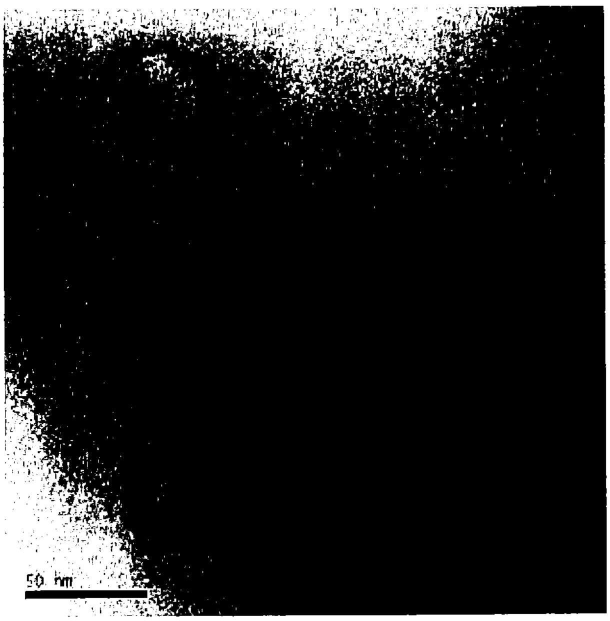

Fig.5

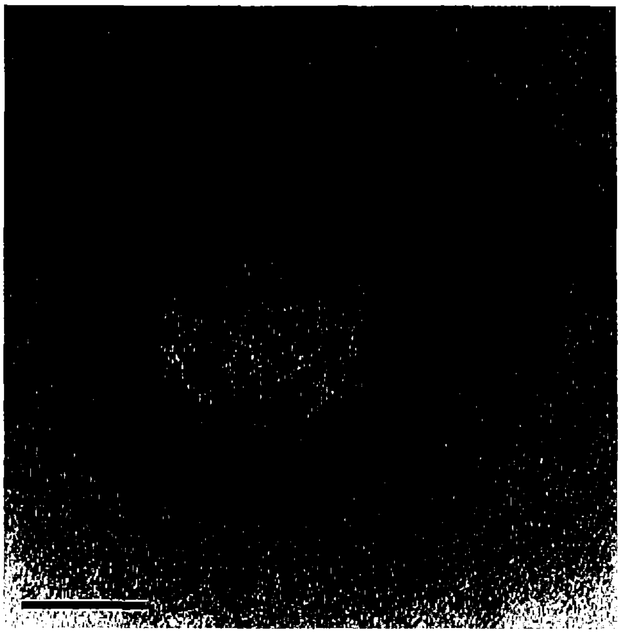

Fig.6

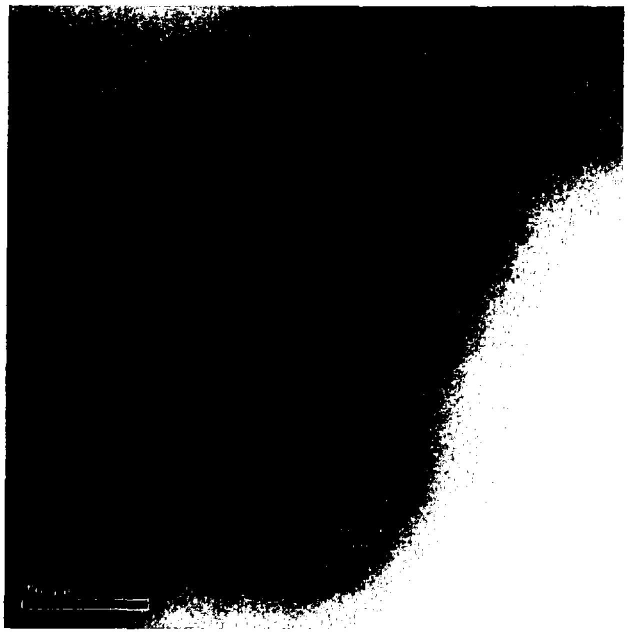

Fig.7

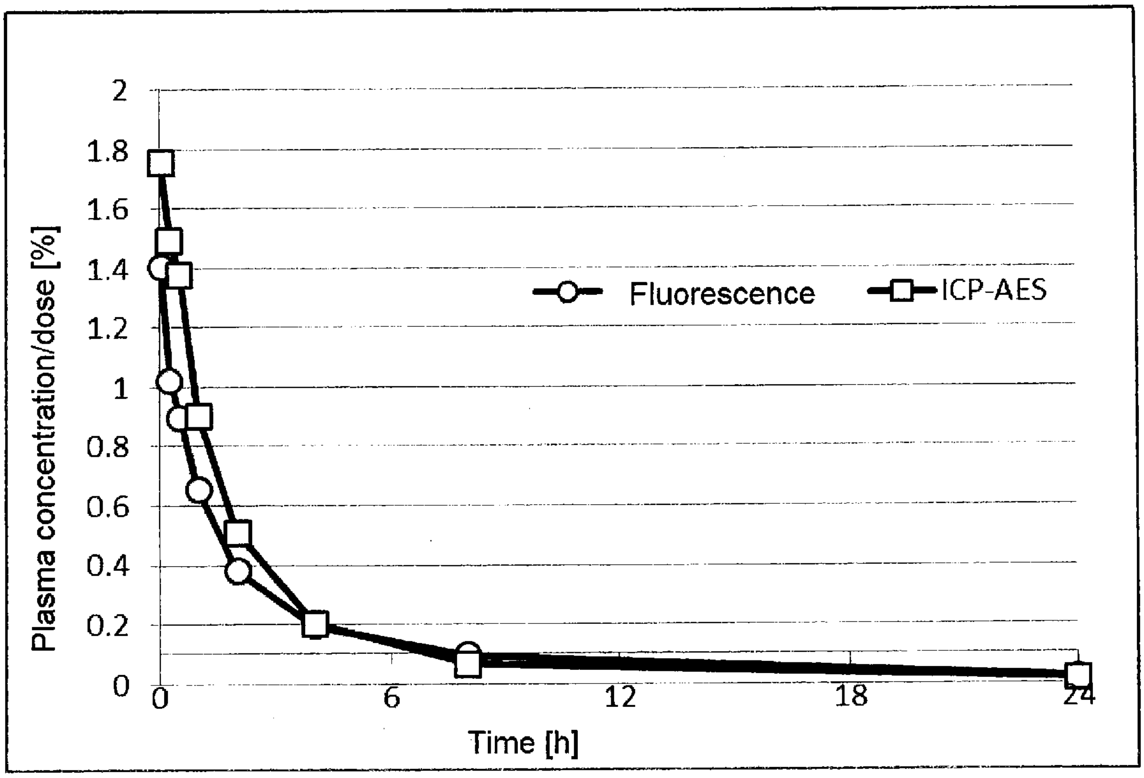
Plasma concentration/dose [%]
2
1.8
1.6
1.4
1.2
1
0.8
0.6
0.4
0.2
0
0
6
12
18
24
Time [h]
Fluorescence
ICP-AES

Fig.8

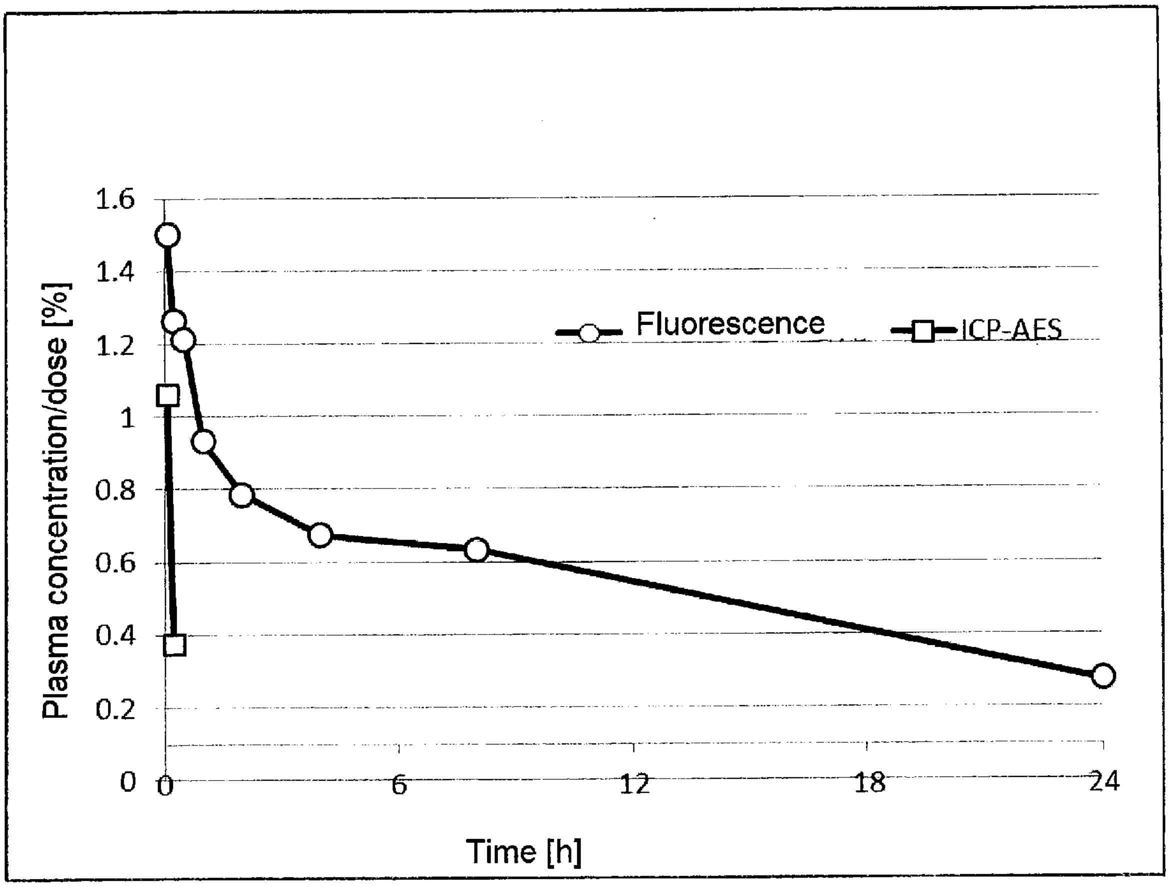
Plasma concentration/dose [%]
1.6
1.4
1.2
1
0.8
0.6
0.4
0.2
0
Fluorescence
ICP-AES
0
6
12
18
24
Time [h]

Fig.9

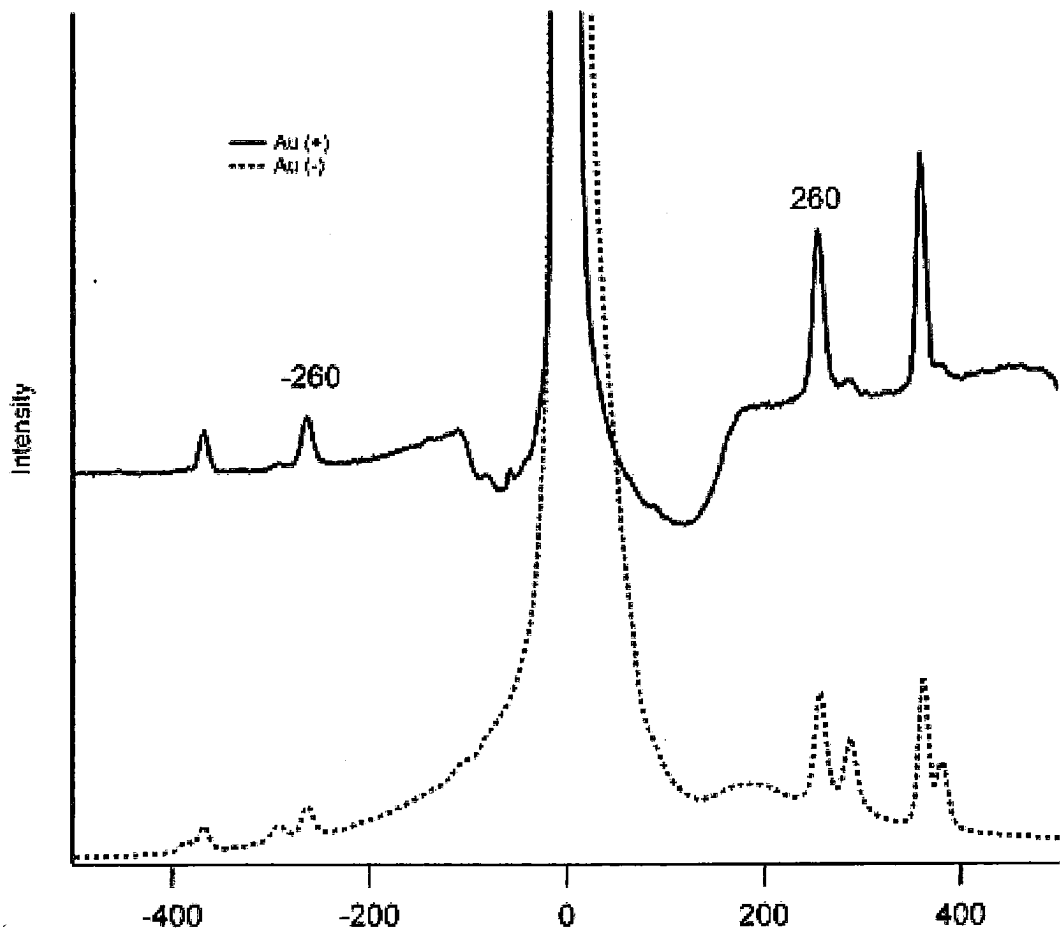

**INTERNATIONAL SEARCH REPORT**

International application No. PCT/JP2013/078400

A. CLASSIFICATION OF SUBJECT MATTER
*A61K9/54*(2006.01)i, *A61K33/24*(2006.01)i, *A61K47/02*(2006.01)i, *A61K47/34*(2006.01)i, *A61K49/00*(2006.01)i, *A61P35/00*(2006.01)i, *A61P43/00*(2006.01)i, *B01J13/14*(2006.01)i, *C08G69/02*(2006.01)i

According to International Patent Classification (IPC) or to both national classification and IPC

B. FIELDS SEARCHED

Minimum documentation searched (classification system followed by classification symbols)
A61K9/54, A61K33/24, A61K47/02, A61K47/34, A61K49/00, A61P35/00, A61P43/00, B01J13/14, C08G69/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2011/145745 A1 (Japan Science and Technology Agency), 24 November 2011 (24.11.2011), entire text & US 2013/0202711 A & EP 2572780 A1 & EP 2626130 A1 & CN 103002982 A | 1-4,7-14<br>5,6 |
| Y | WO 2012/014942 A1 (The University of Tokyo), 02 February 2012 (02.02.2012), entire text & US 2013/0122103 A1 & EP 2599834 A1 | 1-14 |
| Y | WO 2009/133867 A1 (Katayama Chemical Industries Co., Ltd.), 05 November 2009 (05.11.2009), entire text (Family: none) | 5,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 January, 2014 (10.01.14) | 21 January, 2014 (21.01.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/078400

C (Continuation). DOCUMENTS CONSIDERED TO BE RELEVANT

| Category* | Citation of document, with indication, where appropriate, of the relevant passages | Relevant to claim No. |
|---|---|---|
| Y | WO 02/28367 A1 (Kyowa Hakko Kogyo Co., Ltd.), 11 April 2002 (11.04.2002), entire text & JP 2009-7378 A & JP 2010-100645 A & US 2004/0022938 A1 & US 2010/0166847 A1 & EP 1323415 A1 & EP 2241308 A2 & DE 60141078 D & CA 2424619 A & CN 1455664 A | 5,6 |
| Y | JP 2001-501615 A (BASF AG.), 06 February 2001 (06.02.2001), entire text & US 6231848 B1 & EP 939641 A & WO 1998/014199 A1 & DE 19640364 A1 & CA 2267305 A & CN 1238695 A | 5,6 |
| Y | JP 2010-53111 A (Kyushu University), 11 March 2010 (11.03.2010), entire text (Family: none) | 5,6 |
| Y | JP 2011-63867 A (Kyushu University), 31 March 2011 (31.03.2011), entire text (Family: none) | 5,6 |
| A | JP 2008-273881 A (Lion Corp.), 13 November 2008 (13.11.2008), entire text (Family: none) | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011145745 A
- WO 2012014942 A
- JP 2010053111 A
- JP 2011063867 A


### Non-patent literature cited in the description


- *J. Am. Chem. Soc.,* 2010, vol. 132 (5), 1631-1636
- *nanomedicine,* 2010, vol. 6 (1), 161-169
- *J. Electron Microsc. (Tokyo),* 2011, vol. 60 (1), 95-99
- **ANRAKU Y et al.** *J. Am. Chem. Soc.,* 2010, vol. 132 (5), 1631-1636